# EUROPEAN PATENT SPECIFICATION

(11) **EP 3 369 339 B1**
(45) Date of publication and mention of the grant of the patent: **19.07.2023**
(21) Application number: 17176168.7
(22) Date of filing: 15.06.2017
(51) Int. Cl.: A46B 11/00, A46B 3/00, A45D 34/04, A61H 7/00, B05B 11/00

(54) **DOSING CONTAINER**
DOSIERBEHÄLTER
RÉCIPIENT DE DOSAGE

(30) Priority: 02.03.2017 ES 201730218
(43) Date of publication of application: 05.09.2018
(73) Proprietor: CAROBE'LS COSMETICS, S.L., 24010 San Andrés del Rabanedo (ES)
(72) Inventor: CABERO FERNÁNDEZ, Juan José, 24010 San Andrés del Rabanedo (ES)
(74) Representative: Manresa Medina, José Manuel

(56) References cited:
- WO-A1-2013/047983

## Description

A dosing container of the type that comprises a reservoir body, wherein a liquid, cream or gel product to be expelled outside is stored, which defines on its top an upper opening, a cover that closes this opening and which comprises a foam-producing pump that ends in a nozzle, comprising a tube that connects the product from inside the body to the foam-producing pump which mixes the product with air to turn it into foam and transfers it to the nozzle and because it comprises a support that is fixed to the end of the nozzle, with silicone tips defining a brush, with orifices that let the product pass throughout the nozzle to the outside, wherein the perimeter tips being rectangular and the interior ones being cylindrical, with the rectangular ones having a thickness of 0.80 - 0.90 mm and the cylindrical ones having diameters of 0.80 - 0.90 mm and the spacing between the tips being between 0.85 mm and 1.20 mm.

### BACKGROUND TO THE INVENTION

Several aerosols are known in the state of the art that dispense liquids to the outside.

Thus, document WO2013/047983 "Pump dispenser having a push head" from 2012 in the name of Apollo Industrial Co., Ltd belongs to the state of the art, which comprises a pump dispenser having a push head which improves the push head through which contents are sprayed, to directly cover the sprayed content on skin or perform cleansing without using the palms. The pump dispenser includes: a circular disk-shaped brush-mount member communicating with a coupling hole at a central portion of a rear surface thereof while the brush-mount member is inserted and coupled into a front end of a spray passage of a head body. The brush-mount member has a recessed content spray space having a plurality of spray holes punched along an edge and guide passages in a front surface, a cover plate disposed to cover a front portion of the brush-mount member. And a brush member is formed of a rubber material including massage protrusions protruding over an entire front surface of the plate part to elastically contact skin.

### BRIEF DESCRIPTION OF THE INVENTION

This application is framed within the sector of devices for dispensing liquids with greater or lesser viscosity, dosing them by mixing said liquid with air to produce foam.

The problem to be resolved by this invention is how to massage the area while using facial tonics, hair tonics, shampoos, gels or any other type of cosmetic liquids or similar substances.

The inventor has resolved the problem by adding a silicone massager support to the dispensing nozzle of the cosmetic foam, gel or liquid. The support comprises a series of tips so that when the product in form of the foam comes out through orifices in the support, it is distributed between the tips and, at the same time this tips massage the skin, the product is spread over said skin, improving cleansing and removal of dirt from the skin, as well as the remains of other cosmetic or makeup products, thus facilitating complete removal.

One object of this invention is a dosing container of the type that comprises a reservoir body, wherein a liquid, cream or gel product to be expelled outside is stored, which defines on its top an upper opening, a cover that closes this opening and which comprises a foam-producing pump that ends in a nozzle, comprising a tube that connects the product from inside the body to the foam-producing pump, which mixes the product with air to turn it into foam and transfers it to the nozzle, characterised in that it comprises a support that is fixed to the end of the nozzle, with silicone tips defining a brush, with orifices that let the product pass throughout the nozzle to the outside, wherein the perimeter tips being rectangular and the interior ones being cylindrical, with the rectangular ones having a thickness of 0.80 - 0.90 mm and the cylindrical ones having diameters of 0.80 - 0.90 mm and the spacing between the tips is between 0,85 mm and 1.20 mm.

### BRIEF DESCRIPTION OF THE DRAWINGS

In order to facilitate the description, this report is accompanied by two sheets of drawings that represent a practical embodiment case, which is cited by way of example and that does not limit the scope of this invention:
- Figure 1 is a side view of the object of this invention, with a covering element and
- Figure 2 is a frontal view.

### SPECIFIC EMBODIMENT OF THIS INVENTION

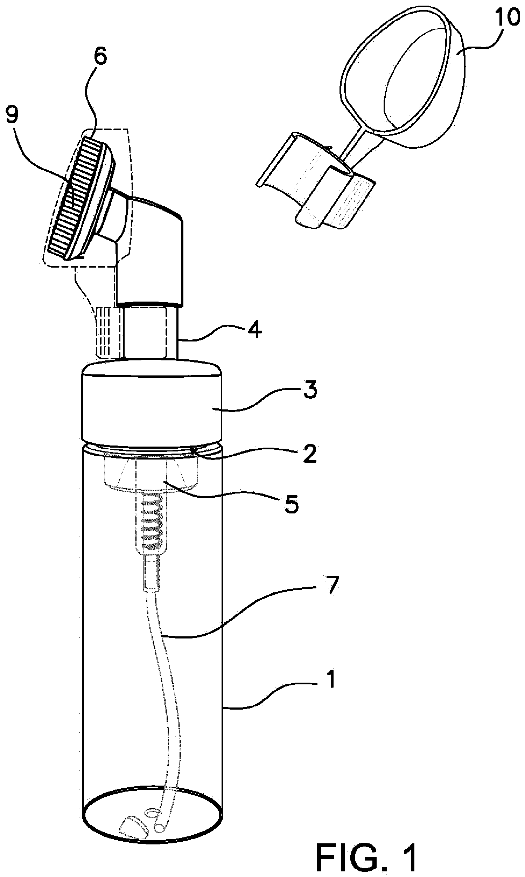

Thus, Figure 1 illustrates body 1, upper opening 2, cover 3, nozzle 4 with its foam-producing pump 5 and its tube 7, support 6 with its tips 9 and covering element 10.

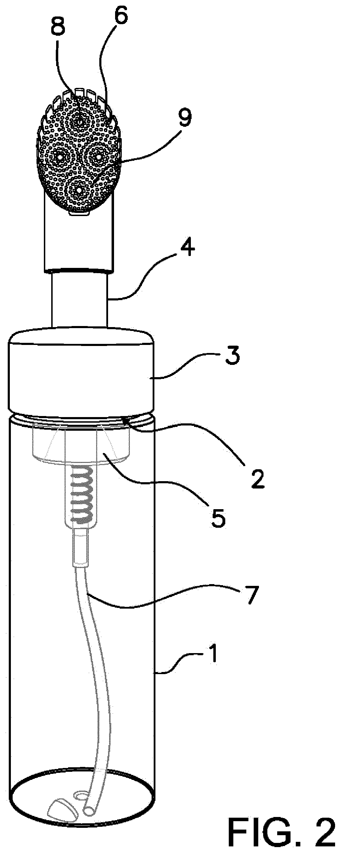

Figure 2 represents body 1, upper opening 2, cover 3, nozzle 4 with its foam-producing pump 5 and its tube 7, support 6 with its tips 9 and orifices 8.

Thus, in a specific exemplary embodiment, the dosing container of this invention comprises body 1 in the form of a reservoir.

In this exemplary embodiment of a cosmetic product, the product, liquid or gel to be mixed into foam is stored inside body 1.

The upper part of body 1 defines an upper opening 2, through which the product to be dispensed is introduced and that is covered by cover 3, which closes off said upper opening 2.

Nozzle 4 is located on said cover 3 and the former is connected to tube 7, which serves as a connection between the product inside body 1 and nozzle 4. A nozzle 4 incorporates foam-producing pump 5 that turns the product into foam and causes it to be expelled outside.

Support 6 is fixed to the end of the nozzle 4, which has tips 9 configured in the form of a brush. Support 6 has orifices 8 for passing the foam product from nozzle 4 to the outside.

In order to provide enhanced sensation on the user's skin, tips 9 are made of silicone, which are soft and also easy to clean and adapt easily to the facial contours.

As it can be seen in Figure 1, tips 9 around the perimeter are rectangular and have a thickness of between 0.80 and 0.90 mm, which the inventor found to be the most suitable for this purpose.

Similarly, the interior tips are cylindrical and have diameters of between 0.80 and 0.90 mm, preferably 0.85 mm. As well as the rectangular ones, the inventor found that this measurement is ideal for the previous purpose and said cylindrical tips 9 are arranged to apply the required pressure over the user's skin in order to clean it, leaving sufficient space for the product to exercise its cleansing action, which will facilitate the cleansing, removal of dirt and the peeling of the skin.

Some tips 9 are separated from contiguous ones by a distance of between 0.85 and 1.20 mm, which defines a space in which part of the foam product is stored for subsequent spreading over the skin.

With this support 6 the dirt in pores is eliminated and the skin looks radiant.

To facilitate the cleaning of support 6, it is provided that support 6 is removable. It would also be an advantage for it to be removable in case of tips 9 being deteriorated because it would mean that one support 6 could be replaced by another without having to discard the container.

In order to prevent the product from drying out and to protect support 6 itself, the possibility is included of a covering element 10 that covers support 6 when not in use (Figure 1).

Thus, in order to use the dispensing container, the user will fill container body 1 by means of upper opening 2 and, once the level is reached, will close said upper opening 2 using cover 3, with tube 7 remaining inside body 1.

The user will then operate pushbutton, which will force the product through tube 7 and nozzle 4 to the end of the nozzle (all this is well-known and is not an object of this invention).

Support 6 is located at the end of nozzle 4. The foam product would leave through orifices 8 and reach support 6, spreading over tips 9 and around them.

The user would position support 6 in contact with the skin and move it massaging the mentioned skin with tips 9 at the same time that the product is being deposited on the skin and is absorbed in a more adequate manner because of the action of tips 9.

As previously indicated, support 6 has a very soft touch and the fact that is has mixed rectangular and cylindrical tips 9 facilitates product application and guarantees skin cleanliness.

This invention describes a new dosing container. The exemplary embodiments mentioned here do not limit this invention and thus, can have various applications and/or adaptations, all of which are within the scope of the following claims.

## Claims

1. Dosing container, of the type that comprises a reservoir body (1), wherein a liquid, cream or gel product is stored to be expelled outside, which defines on its top an upper opening (2), a cover (3) that closes said upper opening (2) and which comprises a foam-producing pump (5) that ends with a nozzle (4), comprising a tube (7) that connects the product from inside the body (1) to the foam-producing pump (5), which mixes the product with air to turn it into foam and transfers it to the nozzle (4), **characterised in that** it comprises a support (6) that is fixed to the end of the nozzle (4), with silicone tips (9) defining a brush, with orifices (8) that let the product pass throughout the nozzle (4) to the outside, wherein the perimeter tips (9) being rectangular and the interior ones being cylindrical, with the rectangular ones having a thickness of 0.80 - 0.90 mm and the cylindrical ones having diameters of 0.80 - 0.90 mm and the spacing between the tips being between 0,85 mm and 1.20 mm.

2. Container in accordance with claim 1, **characterised in that** support (6) is removable.

3. Container in accordance with claim 1, **characterised in that** cylindrical tips (9) have a diameter of 0.85 mm.

4. Container in accordance with any of the previous claims, **characterised in that** it comprises a covering element (10) that covers support (6).

## Patentansprüche

1. Dosierbehälter des Typs, der aus einem Behälter (1) besteht, in dem ein flüssiges, cremiges oder gelartiges Produkt gelagert wird, das nach außen abgegeben werden soll, und an der Oberseite mit einer Öffnung (2) und einem Deckel (3) ausgestattet ist, der die obere Öffnung (2) verschließt. An den Behälter ist eine schaumerzeugende Pumpe (5) angeschlossen, die am Ende eine Düse (4) hat. Diese besteht aus einem Schlauch (7), der das Produkt vom Inneren des Behälters (1) mit der schaumerzeugenden Pumpe (5) verbindet. Diese Pumpe mischt das Produkt mit Luft, das dann zu Schaum wird und leitet es zur Düse (4), die **dadurch gekennzeichnet ist, dass** am Ende der Düse (4) eine Halterung (6) mit Siliziumspitzen (9) in Form einer Büste mit Öffnungen (8) befestigt ist und das Produkt durch die Düse (4) nach außen abgegeben wird. Die äußeren Spitzen sind dabei (9) rechteckig sind und die inneren zylinderförmig, die rechteckigen haben einen Durchmesser von 0,80 - 0,90 mm und die zylinderförmigen 0,80 - 0,90 mm und der Abstand zwischen den Spitzen beträgt zwischen 0,85 mm und 1,20 mm.

2. Behälter nach Anspruch 1, **dadurch gekennzeichnet, dass** die Halterung (6) abnehmbar ist.

3. Behälter nach Anspruch 1, **dadurch gekennzeichnet, dass** die zylinderförmigen Spitzen (9) einen Durchmesser von 0,85 mm haben.

4. Behälter nach einem der vorhergehenden Ansprüche, **dadurch gekennzeichnet, dass** die Halterung (6) mit einer Abdeckung (10) ausgestattet ist.

## Revendications

1. Récipient de dosage du type qui comprend un corps de réservoir (1) dans lequel un liquide, une crème ou un gel est stocké pour être expulsé, qui possède à son sommet une ouverture supérieure (2), un couvercle (3) qui ferme ladite ouverture supérieure (2) et qui comprend une pompe à mousse (5) qui se termine par une buse (4), comprenant un tube (7) qui connecte le produit à l'intérieur du corps (1) à la pompe à mousse (5), laquelle mélange le produit avec de l'air pour le transformer en mousse et le transfère à la buse (4), **caractérisé en ce qu'**il comprend un support (6) qui est fixé à l'extrémité de la buse (4), avec des pointes de silicone (9) formant une brosse, avec des orifices (8) qui laissent passer le produit à travers la buse (4) vers l'extérieur, les pointes périphériques (9) étant rectangulaires et celles de l'intérieur étant cylindriques, les pointes rectangulaires ayant une épaisseur de 0,80 à 0,90 mm et les pointes cylindriques ayant un diamètre de 0,80 à 0,90 mm et l'espacement entre les pointes étant de 0,85 mm à 1,20 mm.

2. Récipient selon la revendication 1, **caractérisé en ce que** le support (6) est amovible.

3. Récipient selon la revendication 1, **caractérisé en ce que** les pointes cylindriques (9) ont un diamètre de 0,85 mm.

4. Récipient selon l'une quelconque des revendications précédentes, **caractérisé en ce qu'**il comprend un élément de couverture (10) qui recouvre le support (6).
